# EUROPEAN PATENT SPECIFICATION

(11) **EP 4 513 144 B1**
(45) Date of publication and mention of the grant of the patent: **14.10.2026**
(21) Application number: 22963194.0
(22) Date of filing: 28.10.2022
(51) Int. Cl.: G01D 3/028, A61B 5/00, G01L 9/00, G01L 19/14

(54) **SENSING APPARATUS**
MESSVORRICHTUNG
APPAREIL DE DÉTECTION

(43) Date of publication of application: 26.02.2025
(73) Proprietor: Shenzhen Shokz Co., Ltd., Shenzhen, Guangdong 518108 (CN)
(72) Inventor: DENG, Wenjun, Shenzhen, Guangdong 518108 (CN); YUAN, Yongshuai, Shenzhen, Guangdong 518108 (CN); HUANG, Yujia, Shenzhen, Guangdong 518108 (CN); ZHOU, Wenbing, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/128401
(87) International publication number: WO 2024/087213

(56) References cited:
- WO-A2-2018/017636
- CN-U- 203 400 138
- CN-U- 206 166 910
- CN-U- 206 166 910
- CN-U- 214 511 151
- CN-U- 214 511 151
- CN-U- 215 677 388
- CN-U- 217 285 764
- US-A- 6 016 704
- US-A1- 2017 095 207
- US-A1- 2017 191 891

## Description

### CROSS-REFERENCE TO RELATED APPLICATIONS

### TECHNICAL FIELD

The present disclosure relates to the field of sensors, and in particular, to sensing devices.

### BACKGROUND

With the development of wearable devices, the requirements for sensors, such as strain sensors, on complex and diversified wearable devices are getting higher. Most traditional strain gauges (also referred to as strain sensors) are designed with a rigid structure, however, rigid strain sensors cannot meet the needs of flexible wearable devices. On the other hand, most existing flexible strain sensors use a capacitive structure or a resistive structure, which may be freely bent and stretched. However, the preparation process of such flexible sensors is complicated, which makes the prepared flexible sensors have a relatively large process deviation. In addition, the flexible sensors are susceptible to the interference of factors such as temperature, humidity, pressure, or the like, which results in poor reliability of the flexible sensors.

A force sensor using an air pressure sensor in a cavity is known from CN214511151U.

### SUMMARY

The invention provides a sensing device comprising a cavity and an air pressure sensor disposed within the cavity. The cavity includes two elastic walls disposed opposite to each other and a rigid wall connecting the two elastic walls, wherein the rigid wall is in a form of a tubular structure, and the two elastic walls are configured to seal the tubular structure to form the cavity. A deformation of the two elastic walls changes air pressure within the cavity, and the air pressure sensor is configured to generate an electrical signal in response to the change of the air pressure.

In some embodiments, a ratio of Young's modulus of the rigid wall to Young's modulus of each of the two elastic walls is greater than 1000.

In some embodiments, a volume of the cavity is in a range of 1 mm³ - 100 mm³.

In some embodiments, the rigid wall includes a first rigid wall and a second rigid wall that are connected to each other, the second rigid wall includes a substrate, and the air pressure sensor is disposed on the second rigid wall.

In some embodiments, the first rigid wall is in a form of an arch or a U-shape, and the first rigid wall is snapped onto the substrate to form the tubular structure.

In some embodiments, Young's modulus of the first rigid wall is greater than 10 Gpa.

In some embodiments, a thickness of the first rigid wall is in a range of 50 um - 300 um.

In some embodiments, the two elastic walls are formed by elastomers that are disposed at two ends of the tubular structure, respectively, and are arranged opposite to each other.

In some embodiments, Young's modulus of each of the elastomers is in a range of 10 KPa-10 MPa.

In some embodiments, a first surface of each of the elastomers is one of a horizontal surface, an inclined surface, or a curved surface.

In some embodiments, a volume of each of the elastomers is in a range of 1 mm³ - 1000 mm³.

In some embodiments, the sensing device further comprises a deformation-limiting portion configured to limit deformation of a second surface of the elastomers in at least one direction, the second surface of the elastomers is in contact with the deformation-limiting portion.

In some embodiments, Young's modulus of the deformation-limiting portion is greater than Young's modulus of each of the elastic walls and is less than Young's modulus of the rigid wall.

In some embodiments, the Young's modulus of the deformation-limiting portion is in a range of 0.5 GPa -10 GPa.

In some embodiments, a thickness of the deformation-limiting portion is in a range of 10 um - 500 um.

In some embodiments, the deformation-limiting portion is configured to limit deformation of the second surface of the elastomers from being stretched or compressed in a first direction; and the deformation-limiting portion extends in an alignment direction of the two elastic walls, the first direction being an extension direction of the deformation-limiting portion.

In some embodiments, the deformation-limiting portion includes a flexible circuit board, the flexible circuit board is disposed on an outer side of a substrate and covers the substrate and the elastomers.

In some embodiments, the sensing device further comprises a flexible circuit board, wherein the flexible circuit board covers an outer side of the rigid wall and does not cover or partially covers a second surface of elastomers.

In some embodiments, the flexible circuit board extends in a direction perpendicular to an alignment direction of the two elastic walls.

In some embodiments, a ratio of an area of the second surface of the elastomers covered by the flexible circuit board to an area of an outer surface of a second rigid wall of the rigid wall is not greater than 2.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering denotes the same structure, wherein:
FIG. 1 is a schematic diagram of an application scenario of a sensing system according to some embodiments of the present disclosure;
FIG. 2A is a schematic diagram of an exemplary structure of a sensing device according to some embodiments of the present disclosure;
FIG. 2B is a schematic diagram of an exemplary cross-section parallel to a YZ plane of the sensing device in FIG. 2A;
FIG. 3A is a schematic diagram of an exemplary cross-section of elastomers according to some embodiments of the present disclosure;
FIG. 3B is a schematic diagram of another exemplary cross-section of elastomers according to some embodiments of the present disclosure;
FIG. 4 is a schematic diagram of an exemplary structure of an air pressure sensor according to some embodiments of the present disclosure;
FIG. 5 is a schematic diagram of bending deformation of a sensing device according to some embodiments of the present disclosure;
FIG. 6 is a curve diagram of an air pressure of a sensing device according to some embodiments of the present disclosure;
FIG. 7A is a schematic diagram of an exemplary structure of a sensing device according to some embodiments of the present disclosure;
FIG. 7B is a schematic diagram of an exemplary cross-section parallel to a YZ plane of the sensing device in FIG. 7A; and
FIG. 8 is a schematic diagram of a stretching deformation or a compression deformation of a sensing device according to some embodiments of the present disclosure.

### DETAILED DESCRIPTION

In order to provide a clearer understanding of the technical solutions of the embodiments described in the present disclosure, a brief introduction to the drawings required in the description of the embodiments is given below. It is evident that the drawings described below are merely some examples or embodiments of the present disclosure, and for those skilled in the art, the present disclosure may be applied to other similar situations without exercising creative labor. Unless otherwise indicated or stated in the context, the same reference numerals in the drawings represent the same structures or operations.

It should be understood that the terms "system," "device," "unit," and/or "module" used herein are ways for distinguishing different levels of components, elements, parts, or assemblies. However, if other terms can achieve the same purpose, they may be used as alternatives.

As indicated in the present disclosure and in the claims, the singular forms "a," "an," and "the" may be intended to include the plural forms as well, unless the context clearly indicates otherwise. In general, the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof.

Flowcharts are used in the present disclosure to illustrate the operations performed by the system according to the embodiments described herein. It should be understood that the operations may not necessarily be performed in the exact sequence depicted. Instead, the operations may be performed in reverse order or concurrently. Additionally, other operations may be added to these processes, or one or more operations may be removed.

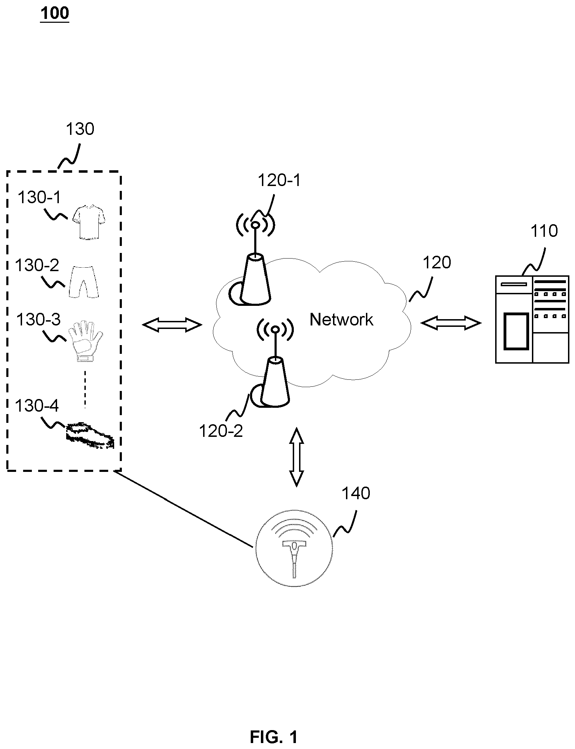

FIG. 1 is a schematic diagram of an application scenario of a sensing system according to some embodiments of the present disclosure. As shown in FIG. 1, a sensing system 100 includes a processing device 110, a network 120, a wearable device 130, and a sensing device 140. In some embodiments, the sensing system 100 may be applied to a variety of scenarios such as pressure measurement, tension measurement, bending measurement, or the like.

In some embodiments, the sensing system 100 may measure a bending condition (e.g., a bending direction and a bending angle) of a body part (e.g., a joint) of a user during movement. In some embodiments, the sensing device 140 may be disposed at a joint portion of the wearable device 130, and when the user wears the wearable device 130 for movement, the movement of the joint of the body of the user may cause the sensing device 140 located at the joint portion to undergo a bending deformation and generate an electrical signal. The processing device 110 may determine a bending condition (e.g., a bending direction and a bending angle) of the sensing device 140 based on the electrical signal generated by the sensing device 140. In some embodiments, the bending direction and the bending angle of the joint when the user moves may be determined based on the bending direction and the bending angle of the sensing device 140. A correspondence between the electrical signal generated by the sensing device 140 and the bending condition of the joint may be established, thereby realizing the bending measurement of the joint. Merely by way of example, the sensing device 140 may be disposed at a wrist joint of the wearable device 130, and bending of the wrist to an inner side and an outer side of an arm may cause the sensing device 140 to generate different electrical signals, based on which the bending direction and the bending angle of the wrist may be determined. In some embodiments, the joint portion may include, but is not limited to, an elbow joint, a knee joint, a wrist joint, a finger joint, a shoulder joint, or the like.

In some embodiments, the sensing system 100 may measure a stretch or compression condition generated by a body part (e.g., a muscle) when the user exercises. In some embodiments, the sensing device 140 may be disposed at a position on the wearable device 130 corresponding to a muscle of the human body, and when the user wears the wearable device 130 for movement, a stretch or compression action of the muscle of the user may cause the sensing device 140 located at the corresponding muscle portion on the wearable device 130 to stretch or compress and generate an electrical signal. The processing device 110 may determine a degree of stretching deformation or compression deformation (e.g., an amount of the stretching deformation or the compression deformation) of the sensing device 140 based on the electrical signal generated by the sensing device 140. In some embodiments, a degree of stretch or compression of the muscle of the user during the exercise may be determined based on the stretching or compression deformation of the sensing device 140. A correspondence between the electrical signal generated by the sensing device 140 and the degree of stretch or compression of the muscle may be established, thereby realizing the stretch or compression measurement of the muscle. In other embodiments, the sensing device 140 may be located at other positions on the wearable device 130 where the stretch or compression condition needs to be measured, and the positions where the sensing device 140 is arranged may be adjusted according to needs.

It should be noted that the sensing system 100 is not limited to the bending measurements and the stretch or compression measurements as described above, but may also be used in other application scenarios, for example, pressure measurements.

In some embodiments, the processing device 110 may be configured to process the electrical signal generated by the sensing device 140. The processing device 110 may receive the electrical signal generated by sensing device 140 and process the electrical signal to determine the degree of the deformation of the sensing device 140. For example, the processing device 110 may determine the bending angle of the sensing device 140 based on the electrical signal generated by the sensing device 140. As another example, the processing device 110 may determine the stretching or compression deformation of the sensing device 140 based on the electrical signal generated by the sensing device 140. In some embodiments, the processing device 110 may also determine whether a movement action of the user is standard or not based on a type of the deformation of the sensing device 140 and a corresponding degree of the deformation. For example, the processing device 110 determines whether pectoralis major muscles of the left and right sides of the user are generating a balanced force when the user performs a chest expansion exercise, whether an angle of a joint movement is within a standard range, or the like.

In some embodiments, the processing device 110 may be local or remote. For example, the processing device 110 may access information stored in the wearable device 130 and/or the sensing device 140 directly or via the network 120. In some embodiments, the processing device 110 may be directly coupled to the wearable device 130 and/or the sensing device 140 to access information stored therein. For example, the processing device 110 may be located in the wearable device 130 and enable information interaction with the sensing device 140 via the network 120. As another example, the processing device 110 may be disposed in the sensing device 140 and enable information interaction with the wearable device 130 via the network 120. In some embodiments, the processing device 110 may execute on a cloud platform. For example, the cloud platform may include a private cloud, a public cloud, a hybrid cloud, a community cloud, a decentralized cloud, an on-premises cloud, etc., or any combination thereof.

In some embodiments, the processing device 110 may include one or more sub-processing devices (e.g., a single-chip processing device or a multi-core multi-chip processing device). Merely by way of example, the processing device 110 includes a central processing unit (CPU), an application-specific integrated circuit (ASIC), an application-specific instruction processor (ASIP), a graphics processor (GPU), a physical processor (PPU), a digital signal processor (DSP), a field programmable gate array (FPGA), a programmable logic circuit (PLD), a controller, a microcontroller unit, a reduced instruction set computer (RISC), a microprocessor, etc., or any combination thereof.

The network 120 may facilitate the exchange of data and/or information between components in the sensing system 100. In some embodiments, one or more components (e.g., the processing device 110, the wearable device 130, and the sensing device 140) of the sensing system 100 may send data and/or information to other components of the sensing system 100 via the network 120. For example, the electrical signal generated by the sensing device 140 may be transmitted to the processing device 110 via the network 120. In some embodiments, the network 120 may be any type of a wired or a wireless network. For example, the network 120 may include a cable network, a wired network, a fiber optic network, a telecommunication network, an intranet network, an inter-network network, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, etc., or any combination thereof. In some embodiments, the network 120 may include one or more network entry and exit points. For example, the network 120 includes wired or wireless network ingress and egress points, such as a base station and/or inter-network switching points 120-1, 120-2, ..., through which the one or more components of the sensing system 100 may be connected to the network 120 to exchange data and/or information.

The wearable device 130 refers to a garment or device that has a wearable function. In some embodiments, the wearable device 130 may include, but is not limited to, a top device 130-1, a pants device 130-2, a glove device 130-3, a shoe 130-4, or the like. In some embodiments, one or more sensing devices 140 may be disposed in the wearable device 130. For example, the one or more sensing device 140 may be provided at one or more of an elbow joint portion, a pectoralis major muscle, and a latissimus dorsi muscle of the top device 130-1, a knee joint portion of the pants device 130-2, a knuckle joint portion of the glove device 130-3, and a foot portion of the shoe 130-4, respectively. When the user wears the wearable device 130 for movement, a movement action of the user may cause a deformation of the sensing device 140 at a corresponding position, the deformation of the sensing device 140 can change air pressure within the sensing device 140, and the sensing device 140 generates an electrical signal in response to the change of the air pressure.

It should be noted that the wearable device 130 is not limited to the top device 130-1, the pants device 130-2, the glove device 130-3, and the shoe device 130-4 as shown in FIG. 1, but may also include other devices applied to other equipment that are required to perform measurements, such as a wristband device, etc. The present disclosure does not impose limitations on the wearable device, and any device that uses the sensing device 140 provided in the present disclosure is within the scope of protection of the present disclosure.

The sensing device 140 may be a sensing device capable of measuring its own deformation. In some embodiments, the sensing device 140 may be disposed on the wearable device 130 (e.g., at a joint portion or a muscle portion). The sensing device 140 is connected (e.g., attached) to the wearable device 130. In some embodiments, the sensing device 140 may include an air pressure sensing device, a deformation of the sensing device 140 generated by an external force causes a change of air pressure within a cavity (e.g., a sealed cavity) where the air pressure sensing device is located, and the air pressure sensing device may generate an electrical signal in response to the change of the air pressure. In some embodiments, the sensing device 140 (e.g., a processor in the sensing device 140) may determine a deformation of the sensing device 140 based on the electrical signal. For example, when the sensing device 140 is disposed at a joint portion on the wearable device 130, a movement of the joint of the user may cause the sensing device 140 to bend and deform, which in turn deforms elastic walls of the cavity of the sensing device 140 and causes a change of air pressure within the cavity. The air pressure sensing device is disposed within the cavity and configured to generate the electrical signal in response to the change of the air pressure. The processor is configured to process the electrical signal to determine the bending direction and the bending angle of the sensing device 140, thereby enabling the measurement of the bending direction and the bending angle of the joint during the movement of the user. As another example, when the sensing device 140 is located at a muscle portion of the wearable device 130, a movement action of the user may cause the sensing device 140 to stretch or compress and deform the cavity of the sensing device 140, thereby causing the elastic walls to deform and causing a change of air pressure within the cavity. The air pressure sensing device generates the electrical signal in response to the change of the air pressure. The processor processes the electrical signal to determine whether the sensing device 140 is stretchedly deformed or compressively deformed and a corresponding amount of the stretching deformation or the compression deformation.

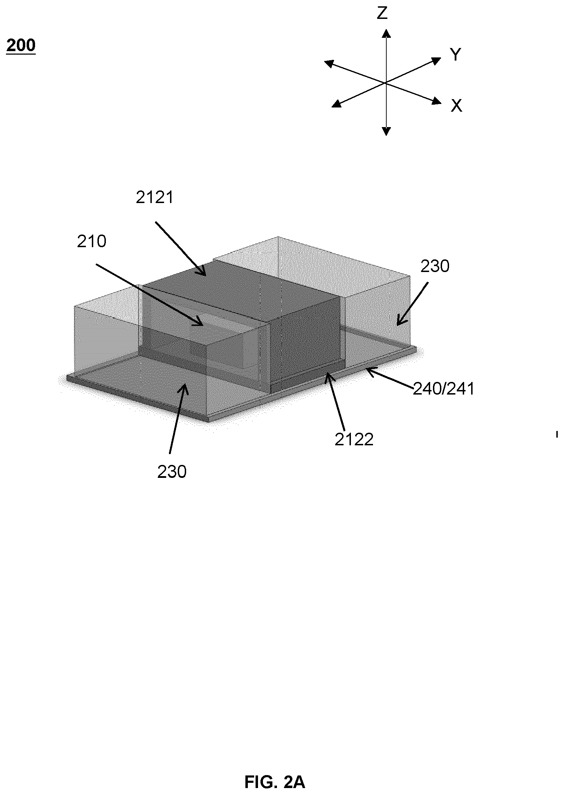

In some embodiments, part of sidewalls of the cavity of the sensing device 140 may be configured as a rigid wall and the remaining sidewalls may be configured as elastic walls (e.g., two elastic walls arranged opposite to each other). When an external force is applied to the sensing device 140, the elastic walls may deform while the rigid wall does not deform, and the deformation of the elastic walls causes a change in a volume of the cavity and a change of air pressure within the cavity, thereby generating the electrical signal. Combining the rigid wall with the elastic walls allows the sensing device 140 to generate an electrical signal only in response to the deformation of the elastic walls, and the force (e.g., the pressure) acting on the rigid wall does not have an effect on the electrical signal output by the sensing device 140, which improves the accuracy of measurements of the sensing device 140. In some embodiments, a bending deformation, a stretching deformation, or a compression deformation of the sensing device 140 under an external force may cause the deformation of the elastic walls. Byconfiguring structures of the sensing device 140 and its components (e.g., a deformation-limiting portion), the sensing device 140 may produce a single type of deformation (e.g., one of the bending deformation, the stretching deformation, or the compression deformation) to allow for the measurement of the type of deformation. For example, when measuring the bending deformation of the sensing device 140, a deformation-limiting portion in the sensing device 140 may be configured to limit the stretching deformation or the compression deformation of elastomers in an extension direction so that the sensing device 140 mainly generates bending deformation and less (even negligible) stretching or compression deformation. In this case, the electrical signal output by the sensing device 140 may characterize a bending direction and a bending angle of the deformation of the sensing device 140. As another example, when measuring the stretching or compression deformation of the sensing device 140, by setting the extension direction and a position of the deformation-limiting portion in the sensing device 140, it is possible to make the deformation-limiting portion less restrictive or non-restrictive of the stretching or compression deformation of the elastomers in the extension direction. At the same time, by adjusting a position (e.g., a non-joint portion) of the sensing device 140 on the wearable device 130, it is possible to make the sensing device 140 mainly generate stretching or compression deformation and less (or even negligible) bending deformation. In this case, the electrical signal output by the sensing device 140 may characterize that the sensing device 140 generates a stretching deformation or a compression deformation and characterize a corresponding deformation degree thereof. In some embodiments, when the sensing device 140 is used for pressure measurement, the pressure measurement may be realized by configuring all of the sidewalls of the cavity of the sensing device 140 as elastic walls (or only one of the sidewalls as a rigid wall, e.g., a substrate as the rigid wall). Further description of the sensing device 140 may be found in FIG. 2A - FIG. 8 of the present disclosure and related descriptions thereof.

In some embodiments, the sensing system 100 may also include other devices or components, e.g., a mobile terminal device, a database, or the like. The mobile terminal device may access information or data in the sensing system 100. In some embodiments, the mobile terminal device may be connected to the wearable device 130 and/or the sensing device 140 via the network 120 (e.g., through a wired connection, a wireless connection, etc.). The user may obtain, via the mobile terminal device, the electrical signal generated by the sensing device 140 during the movement of the user, which may be transmitted via the mobile terminal device to the processing device 110. In some embodiments, the mobile terminal device may include one of a mobile device, a tablet, a laptop, etc., or any combination thereof. In some embodiments, the mobile device may include a cellular phone, a smart home device, a smart mobility device, a virtual reality device, an augmented reality device, or the like, or any combination thereof. In some embodiments, the smart home device may include a control device for a smart appliance, a smart monitoring device, a smart TV, a smart camera, or the like, or any combination thereof. In some embodiments, the smart mobility device may include a smart phone, a personal digital assistant (PDA), a gaming device, a navigation device, a POS device, etc., or any combination thereof. In some embodiments, the virtual reality device and/or the augmented reality device may include a virtual reality helmet, virtual reality glasses, a virtual reality eye mask, an augmented reality helmet, augmented reality glasses, an augmented reality eye mask, etc., or any combination thereof.

The database may store data, such as, the electrical signal generated by the sensing device 140 and sample data on the degree of deformation of the sensing device 140 corresponding to the electrical signal. In some embodiments, the database may store information obtained from the wearable device 130 and/or the mobile terminal device. In some embodiments, the database may include mass memory, removable memory, volatile read-write memory (e.g., random access memory RAM), read-only memory (ROM), etc., or any combination thereof. In some embodiments, the database may be connected to the network 120 to communicate with one or more components (e.g., the processing device 110, the wearable device 130, the sensing device 140, the mobile terminal device, etc.) of the sensing system 100. One or more of the components of the sensing system 100 may access data stored in the database via the network 120. In some embodiments, the database may be part of the processing device 110.

The description of the sensing system 100 is intended to be illustrative and is not intended to limit the scope of the present disclosure. Numerous substitutions, modifications, and variations are apparent to those of ordinary skill in the art. It may be understood that to one of ordinary skill in the art, knowing the principle of the system, it may be possible, without departing from this principle, to make any combination of modules or to form subsystems to connect to other modules. The features, structures, methods, and other characteristics of the exemplary embodiments described in the present disclosure may be combined in various ways to obtain additional and/or alternative exemplary embodiments. For example, the processing device 140 may be integrated into a single device. Additionally, the sensing system 100 may be used for other types of deformation measurements, such as pressure measurements. Modifications such as these are within the scope of protection of the present disclosure.

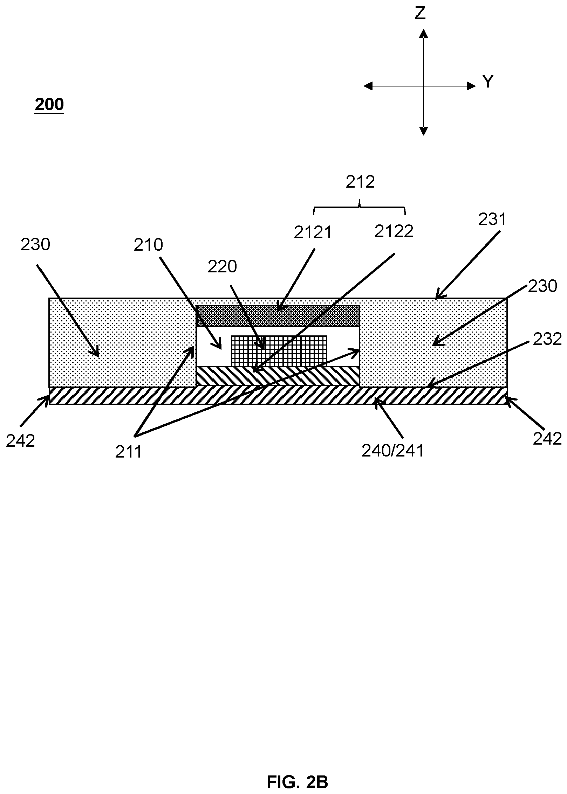

FIG. 2A is a schematic diagram of an exemplary structure diagram of a sensing device according to some embodiments of the present disclosure. FIG. 2B is a schematic diagram of an exemplary cross-section parallel to a YZ plane of the sensing device in FIG. 2A.

As shown in FIG. 2A and FIG. 2B, a sensing device 200 includes a cavity 210 and an air pressure sensor 220 disposed within the cavity 210. The cavity 210 includes two elastic walls 211 disposed opposite to each other and a rigid wall 212 connecting the two elastic walls 211. The rigid wall 212 is in a form of a tubular structure, and the two elastic walls 211 are configured to seal the tubular structure to form the cavity 210. When an external force is applied to the sensing device 200, the two elastic walls 211 are deformed to generate a change of air pressure within the cavity 210, and the air pressure sensor 220 generates an electrical signal in response to the change of the air pressure.

In some embodiments, the rigid wall 212 is in the form of the tubular structure that is internally hollow and has an opening at each of two ends thereof. The two elastic walls 211 are disposed at the two ends of the tubular structure, respectively, to seal the two openings, such that internal space of the tubular structure may form a sealed cavity, i.e., the cavity 210. For example, two elastomers 230 are disposed at the two ends of the tubular structure, and the two elastomers 230 are connected to the two ends of the tubular structure to seal the two openings. A side of each of the two elastomers 230 connected to the tubular structure may be regarded as an elastic wall 211 of the cavity 210. In some embodiments, a shape of the tubular structure may include regular and/or irregular geometric structures such as a hollow cylindrical structure, a semi-cylindrical structure, a square cylindrical structure, or the like.

In some embodiments, when an external force is applied to the sensing device 200, the rigid wall 212 may not deform (or the degree of deformation is so small that it may be ignored), while the elastic walls 211 deform. For example, the elastic walls 211 protrude in a direction toward the cavity 210, or are recessed in a direction away from the cavity 210. The deformation of the elastic walls 211 may cause a change in a volume of the cavity 210, which may cause a change of air pressure within the cavity 210. In some embodiments, when the elastic walls 211 protrude in the direction toward the cavity 210, the volume of the cavity 210 decreases, and the air pressure within the cavity 210 becomes larger; and when the elastic walls 211 are recessed in the direction away from the cavity 210, the cavity 210 becomes larger, and the air pressure within the cavity 210 becomes smaller. The air pressure sensor 220 responds to a change of the air pressure within the cavity 210 and converts the change of the air pressure into a corresponding electrical signal. In some embodiments, a deformation state and a degree of deformation of the elastic walls 211 may be determined based on the electrical signal output by the air pressure sensor 220. For example, if the electrical signal output by the air pressure sensor 220 increases, it may indicate that the elastic walls 211 protrude in the direction toward the cavity 210 to cause the volume of the cavity 210 to decrease and the air pressure to increase. In addition, the greater the amount of change of the electrical signal output by the air pressure sensor 220, i.e., the greater the amount of increase in the electrical signal is, the greater the degree of protrusion of the elastic walls 211 is. As another example, if the electrical signal output by the air pressure sensor 220 decreases, it may indicate that the elastic walls 211 are recessed in the direction away from the cavity 210 to cause the volume of the cavity 210 to increase and the air pressure to decrease, and the greater the amount of change in the electrical signal output by the 220, i.e., the greater the amount of decrease in the electrical signal is, the greater the degree of recession of the elastic walls 211 is.

The air pressure sensor 220 is disposed within the cavity 210, part of sidewalls of the cavity 210 is configured as the rigid wall 212, and remaining sidewalls are configured as two elastic walls 211 that are disposed on two opposite sides of the air pressure sensor 220. This configuration ensures that the air pressure sensor 220 only responds to the change of the air pressure within the cavity 210 caused by a deformation of the elastic walls 211, thus avoiding an influence of an external environmental factor (e.g., a pressure acting on the rigid wall 212) on the electrical signal output by the air pressure sensor 220. In some embodiments, the air pressure sensor 220 is disposed within the cavity 210, and the rigid wall 212 is not deformed under an external force (e.g., a pressure), and the rigid wall 212 plays a protective role for the air pressure sensor 220 and its internal components (e.g., a sensing component and a processing circuit), thereby preventing damage to the air pressure sensor 220 and its internal components, and improving service life of the sensing device 200.

In some embodiments, when an external force acts on the sensing device 200 and causes the elastic walls 211 to deform, in order to ensure that the elastic walls 211 have a better deformation capability and to reduce an effect of the deformation of the rigid wall 212 on the output of the air pressure sensor 220, Young's modulus of each of the two elastic walls and Young's modulus of the rigid wall 212 may be reasonably set. In some embodiments, the Young's modulus of the rigid wall 212 may be set to be greater than the Young's modulus of each of the two elastic walls 211. In some embodiments, a ratio of the Young's modulus of the rigid wall 212 to the Young's modulus of each of the two elastic walls 211 may be greater than 1000. In some embodiments, the ratio of the Young's modulus of the rigid wall 212 to the Young's modulus of each of the two elastic walls 211 may be greater than 1500. In some embodiments, the ratio of the Young's modulus of the rigid wall 212 to the Young's modulus of each of the two elastic walls 211 may be greater than 2000. In some embodiments, the ratio of the Young's modulus of the rigid wall 212 to the Young's modulus of each of the two elastic walls 211 may be greater than 2500. In some embodiments, the ratio of the Young's modulus of the rigid wall 212 to the Young's modulus of each of the two elastic walls 211 may be greater than 3000. In some embodiments, the ratio of the Young's modulus of the rigid wall 212 to the Young's modulus of each of the two elastic walls 211 may be greater than 3500. In some embodiments, the ratio of the Young's modulus of the rigid wall 212 to the Young's modulus of each of the two elastic walls 211 may be greater than 4000. In some embodiments, the ratio of the Young's modulus of the rigid wall 212 to the Young's modulus of each of the two elastic walls 211 may be greater than 4500. In some embodiments, the ratio of the Young's modulus of the rigid wall 212 to the Young's modulus of each of the two elastic walls 211 may be greater than 5000.

By reasonably setting the Young's modulus of the elastic walls 211 and the Young's modulus of the rigid wall 212, it is possible to ensure that the elastic walls 211 can deform in response to the action of an external force while also reducing the effect of the deformation of the rigid wall 212 on the electrical signal output by the air pressure sensor 220, thereby improving accuracy of a measurement result (e.g., a bending direction and a bending angle) of the sensing device 200.

The cavity 210 houses the air pressure sensor 220, and the deformation of the elastic walls 211 causes a change in the volume of the cavity 210, which causes a change of the air pressure within the cavity 210, and the air pressure sensor 220 is configured to generate an electrical signal in response to the change of the air pressure change within the cavity 210. If other conditions (e.g., temperature) are the same, it is known from the above description that for the air within the cavity 210, the air pressure within the cavity 210 may be inversely proportional to the volume of the cavity 210. Therefore, it may be seen that the volume of the cavity 210 may affect a sensitivity of the air pressure sensor 220. With the same degree of deformation of the elastic walls 211, the smaller the volume of the cavity 210 is, the larger the amount of change of the air pressure within the cavity 210 is, and the higher the sensitivity of the air pressure sensor 220 is. In some embodiments, in order to increase the sensitivity of the sensing device 200, the volume of the cavity 210 may be in a range of 1 mm³-100 mm³. In some embodiments, to increase the sensitivity of the sensing device 200, the volume of the cavity 210 may be in a range of 10 mm³-90 mm³. In some embodiments, to increase the sensitivity of the sensing device 200, the volume of the cavity 210 may be in a range of 20 mm³-80 mm³. In some embodiments, to increase the sensitivity of the sensing device 200, the volume of the cavity 210 may be in a range of 30 mm³-70 mm³. In some embodiments, to increase the sensitivity of the sensing device 200, the volume of the cavity 210 may be in a range of 40 mm³-60 mm³. It may be understood that the volume of the cavity 210 refers to an initial volume of the cavity 210, i.e., a volume of the cavity 210 when the elastic walls 211 are not deformed.

The deformation of the elastic walls 211 causes a change in the volume of the cavity 210, and an amount of change in the volume of the cavity 210 caused by the deformation of the elastic wall 211 may be in a range of -10% to 10%. In some embodiments, the amount of change in the volume of the cavity 210 caused by the deformation of the elastic walls 211 may be in a range of -8% to 8%. In some embodiments, the amount of change in the volume of the cavity 210 caused by the deformation of the elastic walls 211 may be in a range of -7% to 7%. In some embodiments, the amount of change in the volume of the cavity 210 caused by the deformation of the elastic walls 211 may be in a range of -6% to 6%. In some embodiments, the amount of change in the volume of the cavity 210 caused by the deformation of the elastic walls 211 may be in a range of -5% to 5%. It should be noted that the amount of change in the volume of the cavity 210 caused by the deformation of the elastic walls 211 is described only as an exemplary description, and in practice, the amount of change in the volume of the cavity 210 may be adjusted by setting a structural parameter (e.g., the volume of the cavity 210) of the sensing device 200 as well as by process optimization.

In some embodiments, the volume of the cavity 210 may be reduced by determining a structure of the elastic walls 211 to increase the sensitivity of the sensing device 200. In some embodiments, each of the elastic walls 211 may be configured as a projecting structure, the projecting structure being a structure of the elastic wall 211 that protrudes in the direction toward the cavity 210 when no external force is applied. With this configuration, the volume of the cavity 210 may be reduced without changing a dimension and a structure of the rigid wall 212, thereby increasing the sensitivity of the sensing device 200. In some embodiments, each of the elastic walls 211 may be configured as the protruding structure in the following manner. The manner includes: preparing the elastomer 230 forming the elastic wall 211 under a condition of a low air pressure (e.g., an air pressure lower than the standard atmospheric pressure), where the elastomer 230 is rendered fluid (e.g., a silicone liquid), the above process being referred to as an injection molding process of the elastomer 230; placing the rigid wall 212 and the air pressure sensor 220 in a fixture that is in an atmospheric pressure environment (e.g., the standard atmospheric pressure environment), and injecting the injection-molded elastomer 230 into the fixture, wherein a change of the air pressure environment may cause a surface of the elastomer 230 that is connected to the rigid wall 212 to penetrate in the direction toward the cavity 210 (or the air pressure sensor 220) and solidify into a shape. By controlling the air pressure during the preparation of the sensing device 200, a morphological structure of the side (i.e., the elastic wall 211) of the elastomer 230 connected to the rigid wall 212 may be adjusted. It should be noted that temperatures of the various operations in the preparation of the sensing device 200 may be maintained consistent, or the morphological structure of the side (i.e., the elastic wall 211) of the elastomer 230 that is connected to the rigid wall 212 may also be further adjusted by controlling the temperatures of the various operations.

In some embodiments, by controlling the degree of protrusion of the elastic walls 211, on the one hand, it is possible to reduce the volume of the cavity 210 and improve the sensitivity of the sensing device 200; on the other hand, it is also possible to prevent the elastic walls 211 from colliding with the sensor when they are deformed. The degree of projection of each of the elastic walls 211 may be expressed as a distance between a vertex of the projection of the elastic wall 211 and a plane in which a sidewall (i.e., a sidewall of the air pressure sensor 220 that is close to the elastic wall 211) of the air pressure sensor 220 corresponding to the elastic wall 211 is located. In some embodiments, the distance between the vertex of the protrusion of the elastic wall 211 and the plane in which the sidewall of the air pressure sensor 220 that is close to the elastic wall 211 is located may be in a range of 1 µm to 1000 µm. In some embodiments, the distance between the vertex of the protrusion of the elastic wall 211 and the plane in which the sidewall of the air pressure sensor 220 that is close to the elastic wall 211 is located may be in a range of 2 µm to 500 µm. In some embodiments, the distance between the vertex of the protrusion of the elastic wall 211 and the plane in which the sidewall of the air pressure sensor 220 that is close to the elastic wall 211 is located may be in a range of 5 µm to 300 µm.

In some embodiments, the volume of the cavity 210 may also be reduced through other ways to increase the sensitivity of the sensing device 200. In some embodiments, the volume of the cavity 210 may be reduced by determining a dimension of the rigid wall 212. For example, the volume of the cavity 210 may be controlled by determining a distance between an inner side of the rigid wall 212 and the air pressure sensor 220.

In some embodiments, the rigid wall 212 may include a first rigid wall 2121 and a second rigid wall 2122 that are connected to each other, the second rigid wall 2122 includes a substrate, and the air pressure sensor 220 is disposed on the substrate. The substrate may be configured to carry the air pressure sensor 220. In some embodiments, the substrate may also be configured to read out the electrical signal generated by the air pressure sensor 220. In some embodiments, the substrate may include a PCB board. In some embodiments, the substrate may also include a substrate made of other materials, such as a ceramic substrate.

In some embodiments, the first rigid wall 2121 may be configured as an arch or a U-shape structure, and the first rigid wall 2121 may snap onto the substrate to form the tubular structure. In some embodiments, the first rigid wall 2121 may be configured as a U-shaped structure, such as a right-angled U-shape structure as shown in FIG. 2A and FIG. 2B. The right-angled U-shape structure snaps onto the substrate to form a square-columnar tubular structure. At this point, the right-angled U-shaped structure forms three sidewalls of the cavity 210, and the substrate forms one sidewall of the cavity 210. That is, the cavity 210 has a total of six sidewalls, wherein the four sidewalls forming the tubular structure (including the three sidewalls of the right-angled U-shaped structure and the substrate) are rigid walls, and the two sidewalls disposed at the two openings on two ends of the tubular structure are elastic walls. In other embodiments, the first rigid wall 2121 may also be a non-right-angled U-shaped structure, for example, a rounded U-shaped structure. The rounded U-shaped structure may avoid a prong of the first rigid wall 2121 from being too sharp, which may result in uncomfortable wearing, thereby improving the wearing comfort of the sensing device 200. In addition, the rounded U-shaped structure reduces stress concentration in and around the prong of the first rigid wall 2121, thereby preventing damage to the sensing device 200. In some embodiments, the first rigid wall 2121 may be in an arched structure, which is snapped onto the substrate to form the tubular structure. Each of the arch structure and the substrate forms a rigid wall of the cavity 210. In some embodiments, the arch structure may include, but is not limited to, a triangular arch structure, a curved arch structure, a trapezoidal arch structure, or the like.

In some embodiments, the first rigid wall 2121 is snapped onto the substrate to form the tubular structure, which on the one hand, can play a protective role for the air pressure sensor 220 disposed in the tubular structure, and on the other hand, can enable the sensing device 200 to be free from the pressure acting on the first rigid wall 2121. In some embodiments, the openings at the two ends of the tubular structure are sealed with the elastomers 230, causing the cavity 210 to have two elastic walls 211, and a deformation (e.g., a bending deformation, a stretching deformation, or a compression deformation) of the elastomers 230 causes the elastic walls 211 to protrude in the direction toward the cavity 210 or to be recessed in the direction away from the cavity 210, thereby causing the change of the air pressure within the cavity 210.

In some embodiments, the first rigid wall 2121 does not deform (or the degree of deformation is so small that it may be ignored) under an action of an external force. Therefore, when applying the sensing device 200 to a wearable device (e.g., the wearable device 130) and taking into account wearing comfort, if a dimension of the first rigid wall 2121 is too large, it may affect the user's wearing experience. Thus, while ensuring that the tubular structure formed by the first rigid wall 2121 and the second rigid wall 2122 is configured to accommodate the air pressure sensor 220, the wearing comfort of the user may be improved by reducing the dimension of the first rigid wall 2121. The tubular structure being configured to accommodate the air pressure sensor 220 means that an inner side of the tubular structure does not collide with the air pressure sensor 220. For example, a width and a length of the first rigid wall 2121 may be equal. The width of the first rigid wall 2121 refers to a dimension along the "X" direction in FIG. 2A, and the length of the first rigid wall 2121 is a dimension along the "Y" direction in FIG. 2A. Additionally, by decreasing the dimension of the first rigid wall 2121, the volume of the cavity 210 is also reduced while the wearing comfort is improved, thereby improving the sensitivity of the sensing device 200.

In some embodiments, in order to ensure that the first rigid wall 2121 does not deform under a pressure, a thickness of the first rigid wall 2121 may be in a range of 50 um-300 um. In some embodiments, the thickness of the first rigid wall 2121 may be in a range of 80 um-280 um. In some embodiments, the thickness of the first rigid wall 2121 may be in a range of 100 um-250 um. In some embodiments, the thickness of the first rigid wall 2121 may be in a range of 120 um-230 um. In some embodiments, the thickness of the first rigid wall 2121 may be in a range of 150 um-200 um. In some embodiments, the thickness of the first rigid wall 2121 may be in a range of 160 um-180 um.

In some embodiments, in order to ensure that the first rigid wall 2121 does not deform under a pressure, Young's modulus of the first rigid wall 2121 may be greater than 10 Gpa. In some embodiments, the Young's modulus of the first rigid wall 2121 may be greater than 50 Gpa. In some embodiments, the Young's modulus of the first rigid wall 2121 may be greater than 150 Gpa. In some embodiments, the Young's modulus of the first rigid wall 2121 may be greater than 200 Gpa. In some embodiments, a material of the first rigid wall 2121 may include a metal (e.g., copper), a metal alloy (e.g., steel), etc. In other embodiments, the material of the first rigid wall 2121 may be a rigid resin material, or the like.

In some embodiments, Young's modulus of the second rigid wall 2122 may be greater than the Young's modulus of the first rigid wall 2121. Alternatively, the Young's modulus of the second rigid wall 2122 may be less than the Young's modulus of the first rigid wall 2121. Parameters such as the Young's modulus and a thickness of the second rigid wall 2122 may be set according to requirements, and the embodiments of the present disclosure do not make specific limitations thereon.

In some embodiments, the first rigid wall 2121 and the second rigid wall 2122 may be two separate structures that are snapped together to form the tubular structure. In some embodiments, the first rigid wall 2121 and the second rigid wall 2122 may be integrally molded.

In some embodiments, the two elastic walls 211 are formed by elastomers 230 that are disposed at two ends of the tubular structure, respectively, and are arranged opposite to each other. In some embodiments, the elastomers 230 may be disposed at the two ends of the tubular structure formed by the rigid wall 212, and the elastomers 230 are connected to the rigid wall 212 and seal the openings of the tubular structure, thereby forming the sealed cavity 210. End surfaces of the elastomers 230 that are connected to the rigid wall 212 may form the elastic walls 211 of the cavity 210. In some embodiments, the elastomers 230 may undergo a deformation, e.g., a bending deformation, a stretching deformation, or a compression deformation, in response to an external force. The deformation of the elastomers 230 may cause a deformation of the elastic walls 211 such that the elastic walls 211 protrude in the direction toward the cavity 210 or are recessed in the direction away from the cavity 210, thereby causing the cavity 210 to change air pressure within the cavity 210.

In some embodiments, as shown in FIGs. 2A and 2B, a first surface 231 of each of the elastomers 230 may cover a top surface (an outer surface of a portion of the first rigid wall 2121 opposite to the second rigid wall 2122) and/or a side surface of the first rigid wall 2121. In this case, the two elastomers 230 may be considered as an integral elastic structure that is encased on an outer peripheral side of the first elastic wall 2121. This arrangement can protect components of the sensing device 200, and at the same time improve the wearing comfort of the user when the sensing device 200 is applied to a wearable device. In some embodiments, the elastomers 230 may also be disposed at the two openings on the two ends of the tubular structure formed by the rigid wall 212. In this case, the two elastomers 230 are two elastic structures that are independent of each other, and the first surfaces 231 of the two elastomers 230 are located in a same plane of the top surface of the first rigid wall 2121. Two surfaces of the two elastomers 230 arranged in the width direction (the "X" direction in FIG. 2A) are respectively located in a same plane as two side surfaces of the first rigid wall 2121 arranged in the width direction. This arrangement can effectively control an overall size of the sensing device 200.

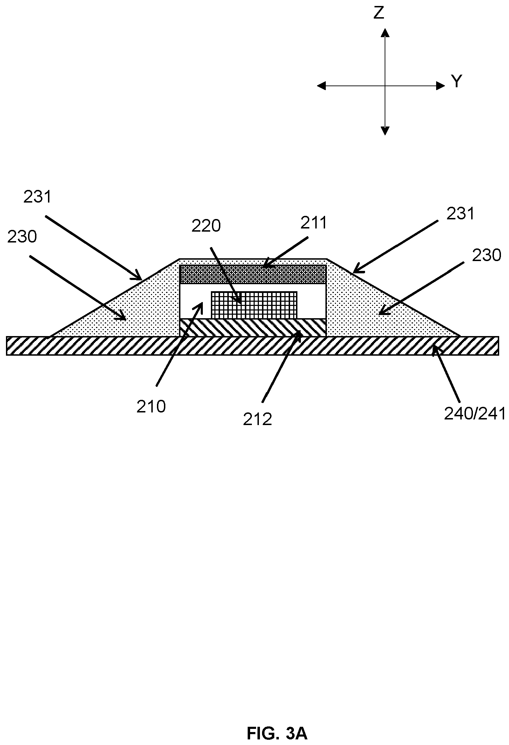

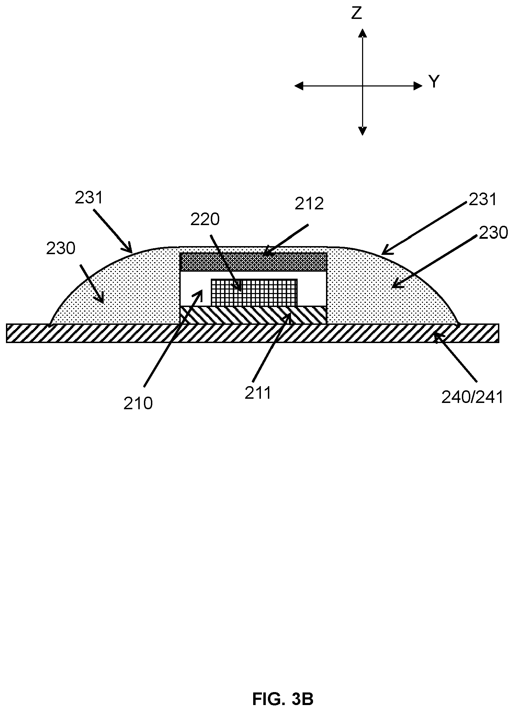

In some embodiments, the first surface 231 of each of the elastomers 230 may be a horizontal surface, as shown in FIG. 2 B. The first surface 231 being a horizontal surface means that the first surface 231 is parallel to a plane in which the second rigid wall 2122 is located. When the first surface 231 of each of the elastomers 230 is a horizontal plane, the elastomers 230 deform better in response to an external force, which allows the sensing device 200 to have a better sensitivity response. In some embodiments, the first surface 231 of each of the elastomers 230 may also be other faceted structures. For example, the first surface 231 of each of the elastomers 230 may be an inclined surface or a curved surface. FIG. 3A is a schematic diagram of an exemplary cross-section of elastomers according to some embodiments of the present disclosure. As shown in FIG. 3A, the first surface 231 of each of the elastomers 230 may be an inclined surface. Along a length direction (the "Y" direction in FIG. 3A), the further the elastomers 230 are from the air pressure sensor 220, the smaller the height (a dimension along the "Z" direction in FIG. 3A) of each of the elastomers 230 is. The elastomer 230 on a single side of the tubular structure may have a triangular or nearly triangular cross-section along a cross-sectional direction A-A. In some embodiments, considering the application of the sensing device 200 in a wearable device, the first surface 231 of each of the elastomers 230 may be configured as a curved surface to improve user experience. FIG. 3B is a schematic diagram of another exemplary cross-section of elastomers according to some embodiments of the present disclosure. As shown in FIG. 3B, the first surface 231 of each of the elastomers 230 is a curved surface, and the height (the dimension along the "Z" direction in FIG. 3B) of each of the elastomer 230 decreases gradually as the elastomer 230 is further away from the air pressure sensor 220.

In some embodiments, the elastomers 230 may deform in response to an external force, and an amount of deformation of the elastomers 230 may affect an amount of change of air pressure within the cavity 210, thereby affecting the electrical signal output by the air pressure sensor 220. Thus, in order to ensure that the air pressure sensor 220 is able to generate the electrical signal in response to the change of air pressure within the cavity 210, so as to improve the accuracy of a measurement result of the sensing device 200, the elastomers 230 need to generate a sufficient amount of deformation in response to the external force, which requires the elastomers 230 to be within a suitable dimension or volume. Additionally, the dimension of the elastomers 230 affects the overall dimension of the sensing device 200, and the dimensions of the sensing device 200 and the elastomers 230 may vary in different application scenarios. For example, when the sensing device 200 is disposed at a knuckle joint and a knee joint of a wearable device to measure a bending angle of the knuckle joint and a bending angle of the knee joint, respectively, the elastomers 230 covering the knuckle joint may have a relatively small dimension, and the elastomers 230 covering the knee joint may have a relatively large dimension. According to the above description, taking into account the amount of deformation of the elastomers 230 and different application scenarios, the volume of a single elastomer 230 may be in a range of 1 mm³ to 1000 mm³. In some embodiments, taking into account the amount of deformation of the elastomers 230 and different application scenarios, the volume of a single elastomer 230 may be in a range of 50 mm³ to 950 mm³. In some embodiments, taking into account the amount of deformation of the elastomers 230 and different application scenarios, the volume of a single elastomer 230 may be in a range of 100 mm³ to 900 mm³. In some embodiments, taking into account the amount of deformation of the elastomers 230 and different application scenarios, the volume of a single elastomer 230 may be in a range of 150 mm³ to 850 mm³. In some embodiments, taking into account the amount of deformation of the elastomers 230 and different application scenarios, the volume of a single elastomer 230 may be in a range of 200 mm³ to 800 mm³. In some embodiments, taking into account the amount of deformation of the elastomers 230 and different application scenarios, the volume of a single elastomer 230 may be in a range of 250 mm³ to 750 mm³. In some embodiments, taking into account the amount of deformation of the elastomers 230 and different application scenarios, the volume of a single elastomer 230 may be in a range of 300 mm³ to 700 mm³. In some embodiments, taking into account the amount of deformation of the elastomers 230 and different application scenarios, the volume of a single elastomer 230 may be in a range of 350 mm³ to 650 mm³. In some embodiments, taking into account the amount of deformation of the elastomers 230 and different application scenarios, the volume of a single elastomer 230 may be in a range of 400 mm³ to 600 mm³. It should be noted that the above ranges of the volume of a single elastomer 230 are only an exemplary description. The volume, length, width, and other dimensions of the elastomers 230 may be adapted according to the application scenarios of the sensing device 200, and the present disclosure does not make any specific limitations in this regard.

In some embodiments, to ensure that the elastomer 230 has a good ability to deform in response to an external force, Young's modulus of each of the elastomers 230 may be in a range of 10 KPa -10 Mpa. In some embodiments, the Young's modulus of each of the elastomers 230 may be in a range of 100 KPa - 9 Mpa. In some embodiments, the Young's modulus of each of the elastomers 230 may be in a range of 200 KPa - 8 Mpa. In some embodiments, the Young's modulus of each of the elastomers 230 may be in a range of 300 KPa -7 Mpa. In some embodiments, the Young's modulus of each of the elastomers 230 may be in a range of 400 KPa - 6 Mpa. In some embodiments, the Young's modulus of each of the elastomers 230 may be in a range of 500 KPa - 5 Mpa. In some embodiments, the Young's modulus of each of the elastomers 230 may be in a range of 600 KPa - 4 Mpa. In some embodiments, the Young's modulus of each of the elastomers 230 may be in a range of 700 KPa - 3 Mpa. In some embodiments, the Young's modulus of each of the elastomers 230 may be in a range of 800 KPa - 2 Mpa. In some embodiments, the Young's modulus of each of the elastomers 230 may be in a range of 900 KPa - 1 Mpa.

In some embodiments, a material of the elastomers 230 may be a material with good elasticity (i.e., susceptible to deformation). In some embodiments, the material of the elastomers 230 may be one or more of a polymer material, a gum material, or the like. In some embodiments, the polymer material may include one of Polydimethylsiloxane (PDMS), Polycarbonate (PC), Polyamides (PA), Acrylonitrile Butadiene Styrene (ABS), Polystyrene (PS), High Impact Polystyrene (HIPS), Polypropylene (PP), Polyethylene Terephthalate (PET), Polyvinyl Chloride (PVC), Polyurethanes (PU), Polyethylene (PE), Phenol Formaldehyde (PF), UreaFormaldehyde (UF), Melamine-Formaldehyde (MF), Polyarylate (PAR), Polyetherimide (PEI), Polyimide (PI), Polyethylene Naphthalate (PEN), Polyetheretherketone (PEEK), silicone, or the like, or any combination thereof.

It should be noted that in other alternative embodiments, only one elastic wall 211 may be disposed in the sensing device 200. For example, the rigid wall 212 may form a tubular structure having an opening at one end, with an elastomer 230 disposed at the opening, and the elastomer 230 seals the opening to form the cavity 210. An end surface of the elastomer 230 connected to the tubular structure forms an elastic wall 211 of the cavity 210. In this arrangement, a deformation (e.g., a bending deformation, a stretching deformation, or a compression deformation) of the elastomer 230 may cause a deformation of the elastic wall 211, which in turn causes a change of air pressure within the cavity 210.

The air pressure sensor 220 is a sensor for detecting air pressure. The air pressure sensor 220 may convert a change of air pressure within the cavity 210 into an electrical signal. For example, a thin film structure may be disposed at an air inlet hole of the air pressure sensor 220, and a change of the air pressure within the cavity 210 deforms the thin film structure, thereby causing a change of air pressure within the air pressure sensor 220, and the air pressure sensor 220 is configured to convert the change of the air pressure within the air pressure sensor 220 into an electrical signal. In some embodiments, the air pressure sensor 220 may include a sensing component (e.g., a MEMS sensor) and the thin film structure. One end of the sensing component may have an opening, and the thin film structure covers the opening. A change of the air pressure within the cavity 210 may cause the thin film structure of the air pressure sensor 220 to deform, thereby causing a change of air pressure within the sensing component, and the sensing component is configured to convert the change of the air pressure therein into an electrical signal. In some embodiments, the air pressure sensor 220 may also include a processor (e.g., an ASIC chip) that matches the MEMS sensor. The processor may process (e.g., filter) the electrical signal generated by the MEMS sensor. In some embodiments, a package manner of the MEMS sensor and the processor may include a top and bottom stacked package or a left and right side-by-side package.

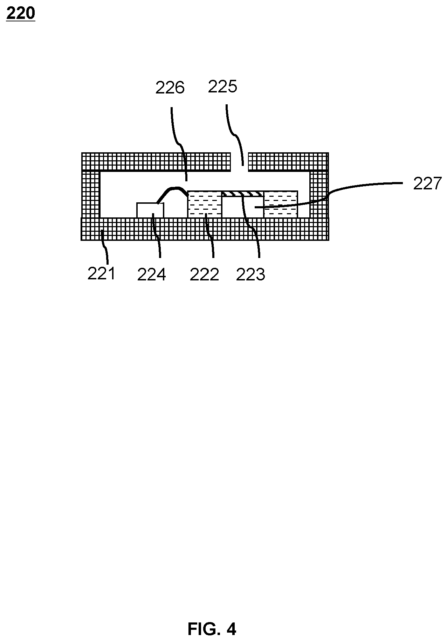

FIG. 4 is a schematic diagram of an exemplary structure of an air pressure sensor according to some embodiments of the present disclosure. As shown in FIG. 4, the air pressure sensor 220 may include a housing structure 221, a membrane structure 223, and a substrate 222. The housing structure 221 is a structural body that is internally hollow, a hollow region of the housing structure 221 is an inner cavity of the housing structure 221, and the membrane structure 223 and the substrate 222 are disposed in the inner cavity. In some embodiments, a shape of the housing structure 221 of the air pressure sensor 220 includes, but is not limited to, a regular shape such as a rectangular body, a sphere, a polygon, a prism, or the like, or any irregular shape. In some embodiments, the substrate 222 is a structural body having an opening at one end, the membrane structure 223 is disposed at the end and covers the opening, and the other end of the substrate 222 is connected to the housing structure 221 to separate the inner cavity into a front cavity 226 and a rear cavity 227. In some embodiments, the substrate 222 may also be a cylindrical structure with two openings at the two ends of the substrate 222, respectively. One end of the substrate 222 is connected to the housing structure 221 and another end of the substrate 222 is connected to the membrane structure 223. The membrane structure 223 seals the opening at the end of the substrate 222 that is connected to the membrane structure 223. In some embodiments, a shape of the opening of the substrate 222 includes, but is not limited to, a regular shape such as a circle, a rectangle, an oval, a semicircle, a polygon, or the like, or any irregular shape. A shape of the membrane structure 223 may include, but is not limited to, a regular shape such as a circle, a rectangle, an ellipse, a semicircle, a polygon, or the like, or any irregular shape. In some embodiments, a dimension of the membrane structure 223 may be larger than a dimension of the openings of the substrate 222 such that the membrane structure 223 seals the opening at the end of the substrate 222 that is connected to the membrane structure 223. In some embodiments, the membrane structure 223 may be connected to a sidewall corresponding to the opening of the substrate 222 by its perimeter, where the membrane structure 223 is adapted to fit the shape and dimension of the opening of the substrate 222.

In some embodiments, an end region of the housing structure 221 of the air pressure sensor 220 may have an hole portion 225, and the hole portion 225 may connect the inner cavity of the air pressure sensor 220 to the cavity 210 of the sensing device 200 when the air pressure sensor 220 is placed in the cavity 210 of the sensing device 200. In some embodiments, the end region of the housing structure 221 in which the hole portion 225 is disposed may be an end region of the housing structure 221 that is located away from the substrate of the sensing device 200, so that the change of the air pressure within the cavity 210 of the sensing device 200 may be received by the air pressure sensor 220 through the hole portion 225.

In some embodiments, the cavity 210 of the sensing device 200 is connected to the front cavity 226 through the hole portion 225, and the air pressure within the cavity 210 may affect air pressure of the front cavity 226 through the hole portion 225, i.e., the change of the air pressure within the cavity 210 causes a change of the air pressure of the front cavity 226. The membrane structure 223 may be configured to deform in response to the change of the air pressure in the front cavity 226. In some embodiments, the sensitivity of the sensing device 200 is correlated with the volume of the cavity 210 and a volume of the front cavity 226 of the air pressure sensor 220. In some embodiments, to increase the sensitivity of the sensing device 200, the volume of the cavity 210 may be not greater than the volume of the front cavity 226 of the air pressure sensor 220. In some embodiments, the sensitivity of the sensing device 200 may be adjusted by adjusting a dimension (e.g., a height, a length, a width, a radius, etc.) of the cavity 210 as well as a dimension of the front cavity 226 and a size of a component (e.g., the substrate 222, the membrane structure 223) within the air pressure sensor 220. It should be noted that the volume of the cavity 210 described in FIG. 4 is a volume of remained space of the cavity after subtracting the space occupied by the air pressure sensor 220.

In some embodiments, the air pressure sensor 220 may further include a first processor 224, the first processor 224 being disposed in the inner cavity of the housing structure 221. The first processor 224 may be communicatively connected to the membrane structure 223 via a lead or in a wireless manner, and the first processor 224 may be configured to perform a signal processing on the electrical signal generated by the air pressure sensor 220 due to a deformation of the membrane structure 223. For example, the first processor 224 may perform an amplification processing, a noise reduction processing, or the like, on the electrical signal.

In some embodiments, a packaging manner for the sensing device 200 may be a primary packaging. A process of the primary packaging is as follows: a bare core (e.g., the air pressure sensor in FIG. 4 that does not include the housing structure 221) of the air pressure sensor 220 is bonded to the substrate, and then the first rigid wall 2121 is snapped to the substrate, further, the snapped structure is placed into a mold and injected with the elastomers 230 to prepare the sensing device 200. At this point, a membrane structure (e.g., the membrane structure 223 in FIG. 4) of the air pressure sensor 220 is directly exposed within the cavity 210 enclosed by the elastomers 230, the first rigid wall 2121, and the substrate. In some embodiments, the packaging manner for the sensing device 200 may be a secondary packaging. A process of the secondary packaging is as follows: first, the air pressure sensor 220 is packaged, and an air inlet hole is provided on the housing structure of the packaged air pressure sensor 220; then, the packaged air pressure sensor 220 is bonded to the substrate, and the first rigid wall 2121 is snapped to the substrate; further, the snapped structure is placed in a mold and injected with elastomers 230 to prepare the sensing device 200.

In some embodiments, an external force acts on the sensing device 200 to deform the elastomers 230, and types of deformations of the elastomers 230 may be different. For example, the elastomers 230 may be configured to generate a bending deformation. Alternatively, the elastomers 230 may be configured to generate a stretching deformation or a compression deformation. Each of the different types of deformations of the elastomers 230 may cause the elastic walls 211 to protrude in the direction toward the cavity 210 or to be recessed in the direction away from the cavity 210, thereby causing a change of air pressure within the cavity 210, and the air pressure sensor 220 generates an electrical signal in response to the change of the air pressure. This configuration makes the sensing device 200 unable to distinguish the types of deformations of the elastomers 230, and the electrical signal output by the air pressure sensor 220 may not accurately measure the degree of deformation of the sensing device 200, which affects the accuracy of the measurement result of the sensing device 200. Therefore, a deformation-limiting portion 240 may be disposed in the sensing device 200, and the deformation-limiting portion 240 is configured to limit the elastomers 230 from generating deformations other than a target deformation, so that a deformation condition of the target deformation of the sensing device 200 may be determined based on the electrical signal output by the air pressure sensor 220. In some embodiments, the target deformation may be a to-be-measured deformation (e.g., a bending deformation) of the sensing device 200. For example, when measuring the bending deformation (i.e., the bending deformation is the target deformation) of the sensing device 200, the deformation-limiting portion 240 may limit the elastomers 230 from being stretched or compressed in an extension direction thereof, so that the elastomers 230 produces only the bending deformation and does not produce stretching deformation or compression deformation (or, the change of the air pressure within the cavity 210 caused by the stretching deformation or compression deformation is so small that it may be ignored). In this case, the bending direction and the bending angle of the sensing device 200 may be determined based on the electrical signal output by the device 200.

In some embodiments, referring to FIG. 2A and FIG. 2B again, the sensing device 200 may include the deformation-limiting portion 240 configured to limit deformation of a second surface 232 of the elastomers 230 in at least one direction. In some embodiments, the deformation-limiting portion 240 is connected to the second surface 232 of the elastomers 230, and the deformation-limiting portion 240 is configured to limit deformation of the second surface 232 of the elastomers 230 from being stretched or compressed in a first direction. It may also be understood that the deformation-limiting portion 240 is configured to restrict the elastomers 230 from generating a stretching deformation or a compression deformation in the first direction. The first direction may be an alignment direction of the two elastomers 230 (or the elastic walls 231), i.e., the Y direction in the drawings. Because the deformation-limiting portion 240 restricts the deformation of the second surface 232 of the elastomers 230 from being stretched or compressed in the first direction, the elastomers 230 only produce the bending deformation under an action of an external force, and the bending direction and the bending angle of the sensing device 200 may be determined based on the electrical signal output by the device 200. In some embodiments, the deformation-limiting portion 240 is configured to extend along the alignment direction of the two elastomeric walls 211, and at this time, the first direction is also the extension direction of the deformation-limiting portion 240. In some embodiments, two extended end surfaces 242 of the deformation-limiting portion 240 may extend beyond a free end surface of each of the elastomers 230. The free end face of the elastomer 230 is an end face of the elastomer 230 that is opposite to the elastic wall 211. It is also understood that a length of the deformation-limiting portion 240 in the extension direction is greater than a distance between the two free end surfaces of the two elastomers 230. In some embodiments, the two extended end surfaces 242 of the deformation-limiting portion 240 may be aligned with the free end surfaces of the elastomers 230 (i.e., as shown in FIG. 2 B, the length of the deformation-limiting portion 240 in the extension direction is equal to the distance between the free end surfaces of the two elastomers 230) or appropriately less than the distance between the free end surfaces of the two elastomers 230 (at which point stretching or compression in the vicinity of the free end surfaces of the elastomers 230 does not result in a change in a shape of the elastic walls 211). In some embodiments, the second surface 232 is disposed opposite to the first surface 231. In some embodiments, the second surface 232 of the elastomers 230 is located in a same plane as a surface (also referred to as a bottom surface of the substrate) of the substrate away from the air pressure sensor 220. The deformation-limiting portion 240 is disposed at a bottom of the sensing device 200, and is affixed to the second surface 232 of the elastomers 230 and the bottom surface of the substrate.

In some embodiments, compared to a scenario when the deformation-limiting portion 240 is not disposed in the sensing device 200, when the deformation-limiting portion 240 is provided to limit the deformation of the second surface 232 of the elastomers 230 from being stretched or compressed in the first direction, the first surface 231 of each of the elastomers 230 may have a greater degree of freedom of deformation, thereby increasing an ability of the elastic walls 211 to deform in response to an external force.

In some embodiments, to ensure that the deformation-limiting portion 240 limits the deformation of the second surface 232 of the elastomers 230 from being stretched or compressed in the first direction without affecting the elastomers 230 from producing a bending deformation, Young's modulus of the deformation-limiting portion 240 may be configured to be greater than the Young's modulus of each of the elastic walls 211 (or the elastomers 230) and less than the Young's modulus of the rigid wall 212. In some embodiments, the Young's modulus of the deformation-limiting portion 240 may be in a range of 0.5 GPa -10 GPa. In some embodiments, the Young's modulus of the deformation-limiting portion 240 may be in a range of 1 GPa-9.5 GPa. In some embodiments, the Young's modulus of the deformation-limiting portion 240 may be in a range of 1.5 GPa - 9 GPa. In some embodiments, the Young's modulus of the deformation-limiting portion 240 may be in a range of 2 GPa - 8.5 GPa. In some embodiments, the Young's modulus of the deformation-limiting portion 240 may be in a range of 2.5 GPa - 8 GPa. In some embodiments, the Young's modulus of the deformation-limiting portion 240 may be in a range of 3 GPa - 7.5 GPa. In some embodiments, the Young's modulus of the deformation-limiting portion 240 may be in a range of 3.5 GPa - 7 GPa. In some embodiments, the Young's modulus of the deformation-limiting portion 240 may be in a range of 4 GPa - 6.5 GPa. In some embodiments, the Young's modulus of the deformation-limiting portion 240 may be in a range of 4.5 GPa - 6 GPa. In some embodiments, the Young's modulus of the deformation-limiting portion 240 may be in a range of 5 GPa - 5.5 GPa.

In some embodiments, a thickness of the deformation-limiting portion 240 may be in a range of 10 um - 500 um. In some embodiments, the thickness of the deformation-limiting portion 240 may be in a range of 50 um - 450 um. In some embodiments, the thickness of the deformation-limiting portion 240 may be in a range of 100 um - 400 um. In some embodiments, the thickness of the deformation-limiting portion 240 may be in a range of 150 um - 350 um. In some embodiments, the thickness of the deformation-limiting portion 240 may be in a range of 200 um - 300 um. In some embodiments, the thickness of the deformation-limiting portion 240 may be in a range of 220 um - 280 um. In some embodiments, the thickness of the deformation-limiting portion 240 may be in a range of 240 um - 260 um.

In some embodiments, the deformation-limiting portion 240 may include a flexible circuit board 241, the flexible circuit board 241 is disposed on an outer side of the substrate (i.e., the bottom surface of the substrate), and covers the substrate and the elastomers 230. In some embodiments, to ensure that the flexible circuit board 241 is able to limit the deformation of the second surface 232 of the elastomers 230 from being stretched or compressed in the first direction, the flexible circuit board 241 may completely cover the bottom surface of the substrate and the second surface 232 of the elastomers 230. In some embodiments, the flexible circuit board 241 may also be configured to solder a lead and read out the electrical signal. In some embodiments, the deformation-limiting portion 240 may also include other structures, for example, flexible films such as polyimide (PI), polyester resin (PET), polyvinyl chloride (PVC), or the like.

It should be noted that the foregoing descriptions of the sensing device 200 and its components are for the purpose of exemplification and illustration only, and do not limit the scope of the present disclosure. For a person skilled in the art, various corrections and changes may be made to the sensing device 200 under the guidance of the present disclosure. These corrections and changes remain within the scope of the present disclosure.

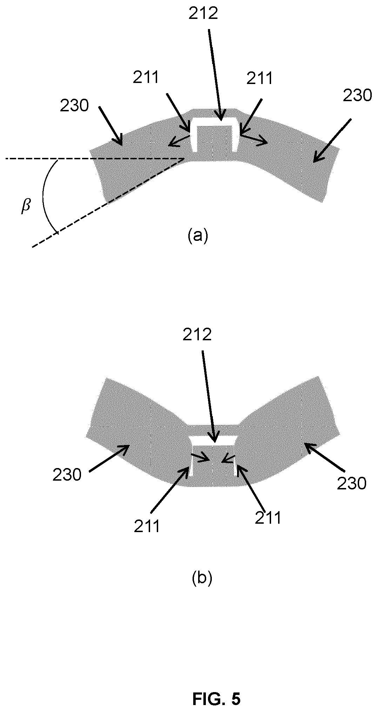

FIG. 5 is a schematic diagram of a bending deformation of a sensing device according to some embodiments of the present disclosure. As may be seen from the above description, when the deformation-limiting portion 240 covers the bottom surface of the substrate and the second surface 232 of the elastomers 230, the deformation-limiting portion 240 may limit deformation of the second surface of the elastomers 230 from being stretched or compressed in the first direction, while causing the elastomers 230 to produce only the bending deformation. At this time, the bending direction and the bending angle of the sensing device 200 may be determined based on the electrical signal output by the air pressure sensor 220. Referring to (a) in FIG. 5, when the sensing device 200 bend upward, i.e., the bending direction of the elastomer 230 is upward bending, the rigid wall 212 does not deform, and the elastomers 230 do not generate the stretching deformation or compression deformation but only generate the bending deformation under the limit of the deformation-limiting portion (not shown in FIG. 5). As the elastomers 230 produce the upward bending deformation, the elastic walls 211 are recessed in a direction away from the cavity, a volume of the cavity increases, and air pressure within the cavity decreases. Referring to (b) in FIG. 5, when the sensing device 200 bends downward, i.e., the bending direction of the elastomers 230 is downward bending, the rigid wall 212 does not deform, the elastic walls 211 protrude in a direction toward the cavity, the volume of the cavity decreases, and the air pressure within the cavity increases. In some embodiments, a change in the electrical signal output by the sensing device 200 may indicate whether the sensing device 200 bends upward or downward. For example, a change in the electrical signal may indicate that the sensing device 200 bends upward. The greater the amount of reduction in the electrical signal is, the greater the upward bending angle of the sensing device 200 is. The smaller the amount of reduction in the electrical signal is, the smaller the upward bending angle of the sensing device 200 is. As another example, increase in the electrical signal indicates that the sensing device 200 bends downward. The larger an amount of increase in the electrical signal is, the larger the upward bending angle of the sensing device 200 is. The smaller the amount of increase in the electrical signal is, the smaller the upward bending angle of the sensing device 200 is. In some embodiments, an angle between a plane in which the second surface 232 of the elastomers 230 is located after the elastomers 230 bend and a plane in which the second surface 232 of the elastomers 230 is located before the elastomers 230 bend may be designated as the bending angle of the sensing device 200. For example, the bending angle of the sensing device 200 may be the angle β in (a) in FIG. 5. The bending angle of the sensing device 200 may also be regarded as a bending angle of the elastomers 230.

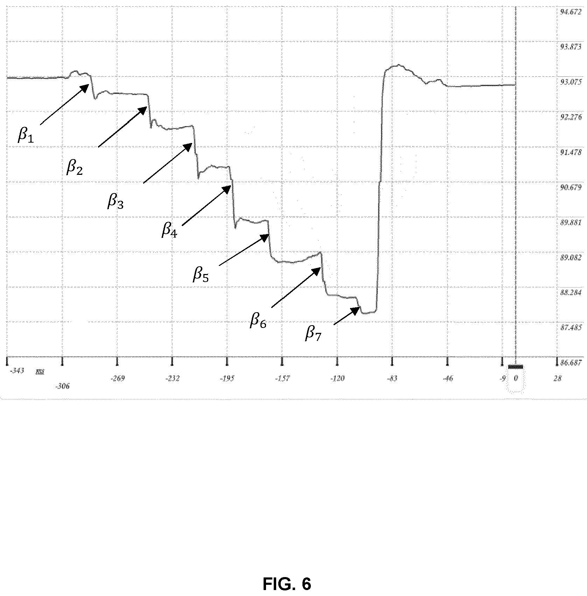

FIG. 6 is a curve diagram of an air pressure of a sensing device according to some embodiments of the present disclosure. In some embodiments, elastomers of the sensing device (e.g., the sensing device 200) undergo varying degrees of bending deformation, e.g., different bending angles of the bending deformation may cause different changes in air pressure within the cavity of the sensing device, and an air pressure sensor of the sensing device may output different air pressure value. That is to say, there is a relationship between the air pressure value of the air pressure sensor and the bending angle of the elastomers. Taking the elastomers bending upward as an example, referring to FIG. 6, the horizontal axis represent the time of an external load acting on the sensing device, unit in ms, the vertical axis the air pressure value of the air pressure sensor, unit in kPa. As may be seen in FIG. 6, a first part (i.e., the left part in FIG. 6) of the air pressure curve of the air pressure sensor exhibits a stepped form, in which the upward bending angle of the elastomers corresponding to each step gradually increases from left to right. When the elastomer bends upward, the air pressure value of the air pressure sensor gradually decreases. The larger the upward bending angle of the elastomers is, the smaller the air pressure value of the air pressure sensor is. The smaller the upward bending angle of the elastomers is, the larger the air pressure value of the air pressure sensor is.

It should be noted that the reason that the first part of the air pressure curve shown in FIG. 6 exhibits the stepped form may be that an upward bending angle of the elastomers in an initial state is 0 degrees, at which time the air pressure value of the air pressure sensor is an initial value of the air pressure. As the external force acts on the sensing device to cause an upward bending angle β₁ of the elastomers, the air pressure value of the air pressure sensor becomes smaller, and the air pressure curve of the air pressure sensor corresponds to a descending edge of a step. The upward bending angle of the elastomer is kept at β₁, and the air pressure value of the air pressure sensor is essentially maintained in the process. Further, the elastomers continue to bend to an upward bending angle β₂ on the basis of the upward bending angle β₁ of the elastomers, during which the air pressure value of the air pressure sensor continues to become smaller, and the air pressure curve of the air pressure sensor corresponds to the descending edge of a next step. In the same manner, the upward bending angle of the elastomers is made to gradually increase in an order of β₃, β₄, β₅, β₆, and β₇ (β₇ > β₆ > β₅ > β₄ > β₃ > β₂ > β₁), thereby exhibiting a plurality of consecutive steps as shown in FIG. 6.

Referring to FIG. 6 again, a second part (i.e., the right part of FIG. 6) of the air pressure curve of the air pressure sensor represents a process in which the upward bending angle of the elastomers is restored from a maximum angle to the initial state (i.e., restoring from the upward bending angle β₇ to 0 degrees). Correspondingly, the air pressure value of the air pressure sensor is restored to the initial air pressure value.

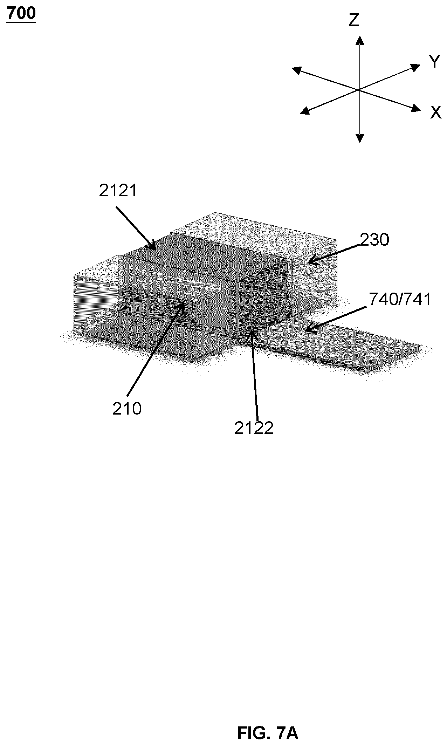

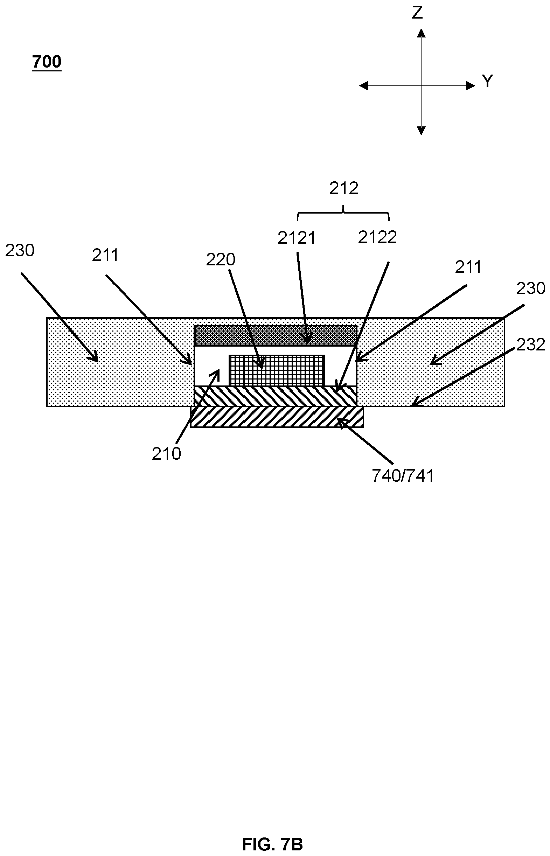

According to the above description, when the deformation-limiting portion covers a large portion of the second surface of the elastomers (i.e., the deformation-limiting portion covers a large coverage area of the second surface of the elastomers), the deformation-limiting portion limits the deformation of the second surface of the elastomers from being stretched or compressed in the first direction. When the deformation-limiting portion covers a small portion (i.e., the deformation-limiting portion covers a small coverage area of the second surface of the elastomers) or when the deformation-limiting portion does not cover the elastomers (in which case the coverage area is zero), then the elastomers may be stretched or compressed in the first direction. Therefore, by adjust an area of the deformation-limiting portion and a position of the deformation-limiting portion in the sensing device, the elastomers may produce stretching deformation or compression deformation under an action of an external force. In this case, it may be determined, based on the electrical signal output by the air pressure sensor, whether a deformation of the sensing device is a stretching deformation or a compression deformation, and an amount of the stretching deformation or the compression deformation. FIG. 7A is a schematic diagram of an exemplary structure of a sensing device according to some embodiments of the present disclosure. FIG. 7B is a schematic diagram of an exemplary cross-section parallel to a YZ plane of the sensing device in FIG. 7A. The structure of a sensing device 700 shown in FIGs. 7A and 7B are the same or substantially the same as the structure of the sensing device 200 shown in FIGs. 2A and 2B, and descriptions of the same or similar structures (e.g., the air pressure sensor 220, the rigid wall 212, the elastic walls 211, the elastomers 230, or the like) may be found in FIGs. 2A and 2B and the related descriptions thereof, which will not be repeated herein. The sensing device 700 differs from the sensing device 200 in that the deformation-limiting portion is provided in a different manner.

Referring to FIG. 7A and FIG. 7B, a deformation-limiting portion 740 of the sensing device 700 may include a flexible circuit board 741. In some embodiments, a flexible circuit board 741 may be disposed on and cover a bottom surface of the substrate and not cover or partially cover the second surface 232 of the elastomers 230. In some embodiments, the flexible circuit board 741, when covering the second surface 232 of the elastomers 230, may have an effect on the stretching deformation or the compression deformation of the second surface 232 of the elastomers 230 in the first direction. Therefore, to minimize the effect of the flexible circuit board 741 on the stretching deformation or the compression deformation of the elastomers 230, an area of the flexible circuit board 741 covering the second surface 232 of the elastomers 230 may be reasonably set. In some embodiments, a ratio of the area of the flexible circuit board 741 covering the second surface 232 of the elastomers 230 to an area of an outer surface of the second rigid wall 2122 (i.e., the bottom surface of the substrate) may not be greater than 2. In some embodiments, the ratio of the area of the flexible circuit board 741 covering the second surface 232 of the elastomers 230 to the area of the outer surface of the second rigid wall 2122 (i.e., the bottom surface of the substrate) may not be greater than 1.8. In some embodiments, the ratio of the area of the flexible circuit board 741 covering the second surface 232 of the elastomers 230 to the area of the outer surface of the second rigid wall 2122 (i.e., the bottom surface of the substrate) may not be greater than 1.5. In some embodiments, the ratio of the area of the flexible circuit board 741 covering the second surface 232 of the elastomers 230 to the area of the outer surface of the second rigid wall 2122 (i.e., the bottom surface of the substrate) may not be greater than 1.3. In some embodiments, the ratio of the area of the flexible circuit board 741 covering the second surface 232 of the elastomers 230 to the area of the outer surface of the second rigid wall 2122 (i.e., the bottom surface of the substrate) may not be greater than 1. In some embodiments, the ratio of the area of the flexible circuit board 741 covering the second surface 232 of the elastomers 230 to the area of the outer surface of the second rigid wall 2122 (i.e., the bottom surface of the substrate) may not be greater than 0.8. In some embodiments, the ratio of the area of the flexible circuit board 741 covering the second surface 232 of the elastomers 230 to the area of the outer surface of the second rigid wall 2122 (i.e., the bottom surface of the substrate) may not be greater than 0.5. In some embodiments, the flexible circuit board 741 may not cover the second surface 232 of the elastomers 230, in which case a length of the flexible circuit board 741 along the Y direction is equal to a length of the substrate along the Y direction.

In some embodiments, the flexible circuit board 741 may be configured to solder a lead and read out the electrical signal. In some embodiments, an overall dimension of the flexible circuit board 741 may be affected when the flexible circuit board 741 does not cover, or partially covers, the second surface 232 of the elastomers 230. In order to ensure that the flexible circuit board 741 has a sufficient dimension for arranging a circuit structure, the flexible circuit board 741 may be configured to extend in a direction perpendicular to an alignment direction of the two elastic walls 211, so that the overall dimension of the flexible circuit board 741 satisfy the needs of circuit soldering. When the flexible circuit board 741 extends toward the direction perpendicular to the alignment direction of the two elastic walls 211, a length of the flexible circuit board 741 along the X direction is greater than a length of the substrate along the X direction. In some embodiments, in order to reduce the overall dimension of the sensing device 200, the length of the flexible circuit board 741 along the X direction may also be set to be equal to the length of the substrate along the X direction. The length of the flexible circuit board 741 along the X direction may be set in consideration of the needs of the sensing device 200, for example, the overall dimension of the sensing device 200, circuit welding, or the like, and the present disclosure does not impose specific limitations.

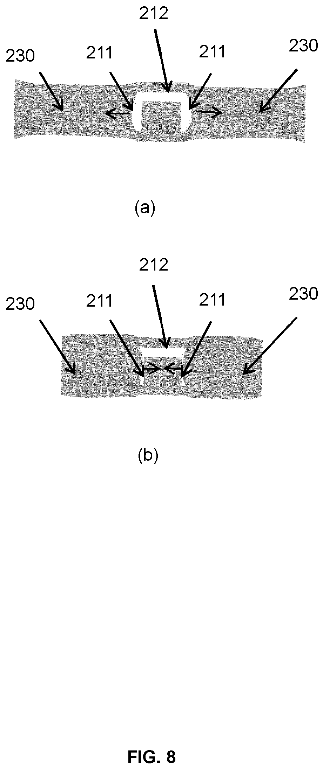

FIG. 8 is a schematic diagram of a stretching deformation or a compression deformation of a sensing device according to some embodiments of the present disclosure. As may be seen from the descriptions of FIG. 7A and FIG. 7B, the elastomers 230 may undergo a stretching deformation or a compression deformation when the flexible circuit board 741 does not cover or partially covers a substrate bottom surface. In this case, it may be determined, based on an electrical signal output by the air pressure sensor 220, whether the sensing device 700 generates a stretching deformation or a compression deformation, and an amount of the stretching deformation or the compression deformation. In some embodiments, two elastomers may have a first distance between two free end surfaces of the two elastomers before being stretched or compressed and a second distance between the two free end surfaces of the two elastomers after being stretched or compressed. An absolute value of a difference between the first distance and the second distance may be designated as the amount of the stretching deformation or the compression deformation. Referring to (a) in FIG. 8, the sensing device (e.g., the sensing device 700) stretches to both sides without deformation of the rigid wall 212 and the elastomer 230 stretches to both sides. As the elastomers 230 is stretched to two sides of the sensing device, the elastic walls 211 are recessed in a direction away from a cavity (e.g., the cavity 210) of the sensing device, a volume of the cavity increases, and the air pressure within the cavity decreases. Referring to (b) in FIG. 8, when the sensing device is compressed toward a center of the sensing device, the rigid wall 212 does not deform, the elastic walls 211 protrude in a direction toward the cavity, the volume of the cavity decreases, and the air pressure within the cavity increases. In some embodiments, a change in the electrical signal output by the sensing device 700 may indicate whether the sensing device 700 generates a stretching deformation or a compression deformation. For example, the change in the electrical signal may indicate that a deformation state of the sensing device 700 is the stretching deformation. The larger the decrease in the electrical signal is, the larger an amount of the stretching deformation of the sensing device 700 is. The smaller the decrease in the electrical signal is, the smaller the amount of the stretching deformation of the sensing device 700 is. As another example, when the electrical signal becomes larger, it may indicate that the deformation state of the sensing device 700 is the compression deformation. The larger the amount of increase in the electrical signal is, the larger the amount of the stretching deformation of the sensing device 700 is. The smaller the amount of increase in the electrical signal is, the smaller the amount of the stretching deformation of the sensing device 700 is.

Having thus described the basic concepts, it may be rather apparent to those skilled in the art after reading this detailed disclosure that the foregoing detailed disclosure is intended to be presented as illustrative example and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of the present disclosure.

Moreover, certain terminology has been configured to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics in one or more embodiments of the present disclosure may be combined as suitable.

Furthermore, it may be understood by those skilled in the art that various aspects of the present disclosure may be explained and described through several patentable types or situations, including any new and useful process, machine, manufacture, or composition of matter, or any new and useful improvement thereof. Accordingly, various aspects of the present disclosure may be implemented entirely by hardware, entirely by software (including firmware, resident software, microcode, etc.), or by a combination of hardware and software. The aforementioned hardware or software may be referred to as "data blocks," "modules," "engines," "units," "components," or "systems." Additionally, aspects of the present disclosure may manifest as a computer product located on one or more computer-readable media, which includes computer-readable program code.

The computer storage medium may contain a propagated data signal with computer program code embedded in it, for example, in baseband or as part of a carrier wave. The propagated signal may take various forms, including an electromagnetic form, an optical form, or suitable combinations thereof. The computer storage medium may be any computer-readable medium other than a computer-readable storage medium, which may be connected to an instruction execution system, apparatus, or device to enable communication, propagation, or transmission of a program for use. The program code stored on the computer storage medium may be propagated via any suitable medium, including wireless, cable, fiber optic cable, RF, or similar media, or any combination of the aforementioned media.

The computer program code required to perform operations of various parts of the present disclosure may be written in any one or more programming languages, including object-oriented programming languages such as Java, Scala, Smalltalk, Eiffel, JADE, Emerald, C++, C#, VB.NET, Python, etc., conventional procedural programming languages such as C, Visual Basic, Fortran 2003, Perl, COBOL 2002, PHP, ABAP, dynamic programming languages such as Python, Ruby, and Groovy, or other programming languages. The program code may run entirely on the user's computer, as a standalone software package on the user's computer, partly on the user's computer and partly on a remote computer, or entirely on a remote computer or processing device. In the latter case, the remote computer may be connected to the user's computer via any type of network, such as a local area network (LAN) or a wide area network (WAN), or the remote computer may be connected to an external computer (e.g., via the internet), or operate in a cloud computing environment, or be used as a service such as Software as a Service (SaaS).

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be noted that in the foregoing description of embodiments of the present disclosure, various features are sometimes grouped together in a single embodiment, figure, or description thereof for the purpose of streamlining the disclosure aiding in the understanding of one or more of the various inventive embodiments. This way of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties configured to describe and claim certain embodiments of the present disclosure are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameter set forth in the written description and attached claims are approximations that may vary depending upon the desired properties sought to be obtained by a particular embodiment. In some embodiments, the numerical parameter should be construed in light of the number of reported significant digits and by applying ordinary rounding techniques. Notwithstanding that the numerical ranges and parameter setting forth the broad scope of some embodiments of the present disclosure are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the present disclosure disclosed herein are illustrating of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A sensing device comprising a cavity (210) and an air pressure sensor (220), wherein the air pressure sensor (220) is disposed within the cavity (210), the sensing device is **characterized in that**:
the cavity (210) includes two elastic walls (211) disposed opposite to each other and a rigid wall (212) connecting the two elastic walls (211), wherein the rigid wall (212) is in a form of a tubular structure, and the two elastic walls (211) are configured to seal the tubular structure to form the cavity (210);
wherein a deformation of the two elastic walls (211) changes air pressure within the cavity (210), and the air pressure sensor (220) is configured to generate an electrical signal in response to the change of the air pressure.

2. The sensing device of claim 1, wherein a ratio of Young's modulus of the rigid wall (212) to Young's modulus of each of the two elastic walls (211) is greater than 1000.

3. The sensing device of claim 1, wherein a volume of the cavity (210) is in a range of 1 mm³ - 100 mm³.

4. The sensing device of claim 1, wherein the rigid wall (212) includes a first rigid wall (2121) and a second rigid wall (2122) that are connected to each other, the second rigid wall (2122) includes a substrate, and the air pressure sensor (220) is disposed on the second rigid wall (2122).

5. The sensing device of claim 4, wherein the first rigid wall (2121) is in a form of an arch or a U-shape, and the first rigid wall (2121) is snapped onto the substrate to form the tubular structure.

6. The sensing device of claim 4, wherein Young's modulus of the first rigid wall (2121) is greater than 10 Gpa.

7. The sensing device of claim 1, wherein the two elastic walls (211) are formed by elastomers that are disposed at two ends of the tubular structure, respectively, and are arranged opposite to each other.

8. The sensing device of claim 7, wherein Young's modulus of each of the elastomers is in a range of 10 KPa-10 MPa.

9. The sensing device of claim 7, further comprising a deformation-limiting portion (240) configured to limit deformation of a second surface (232) of the elastomers in at least one direction, the second surface (232) of the elastomers is in contact with the deformation-limiting portion (240).

10. The sensing device of claim 9, wherein Young's modulus of the deformation-limiting portion (240) is greater than Young's modulus of each of the elastic walls (211) and is less than Young's modulus of the rigid wall.

11. The sensing device of claim 10, wherein the Young's modulus of the deformation-limiting portion (240) is in a range of 0.5 GPa -10 GPa.

12. The sensing device of claim 9, wherein the deformation-limiting portion (240) is configured to limit deformation of the second surface (232) of the elastomers from being stretched or compressed in a first direction; and the deformation-limiting portion (240) extends in an alignment direction of the two elastic walls (211), the first direction being an extension direction of the deformation-limiting portion (240).

13. The sensing device of any one of claims 9-12, wherein the deformation-limiting portion (240) includes a flexible circuit board (241), the flexible circuit board (241) is disposed on an outer side of a substrate and covers the substrate and the elastomers.

14. The sensing device of any one of claims 1-8, further comprising a flexible circuit board (241), wherein the flexible circuit board (241) covers an outer side of the rigid wall and does not cover or partially covers a second surface (232) of elastomers.

15. The sensing device of claim 14, wherein the flexible circuit board (241) extends in a direction perpendicular to an alignment direction of the two elastic walls (211).

## Patentansprüche

1. Sensorvorrichtung, die einen Hohlraum (210) und einen Luftdrucksensor (220) umfasst, wobei der Luftdrucksensor (220) innerhalb des Hohlraums (210) angeordnet ist,
wobei die Sensorvorrichtung **dadurch gekennzeichnet ist, dass**:
der Hohlraum (210) zwei einander gegenüberliegend angeordnete elastische Wände (211) und eine starre Wand (212), die die zwei elastischen Wände (211) verbindet, einschließt, wobei die starre Wand (212) in der Form einer rohrförmigen Struktur ist und die zwei elastischen Wände (211) so konfiguriert sind, dass sie die rohrförmige Struktur abdichten, um den Hohlraum (210) bilden;
wobei eine Verformung der zwei elastischen Wände (211) Luftdruck innerhalb des Hohlraums (210) verändert und der Luftdrucksensor (220) so konfiguriert ist, dass er als Reaktion auf die Änderung des Luftdrucks ein elektrisches Signal erzeugt.

2. Sensorvorrichtung nach Anspruch 1, wobei ein Verhältnis von Elastizitätsmoduls der starren Wand (212) zum Elastizitätsmodul jeder der zwei elastischen Wände (211) größer als 1000 ist.

3. Sensorvorrichtung nach Anspruch 1, wobei ein Volumen des Hohlraums (210) im Bereich von 1 mm³ - 100 mm³ liegt.

4. Sensorvorrichtung nach Anspruch 1, wobei die starre Wand (212) eine erste starre Wand (2121) und eine zweite starre Wand (2122) einschließt, die miteinander verbunden sind, wobei die zweite starre Wand (2122) ein Substrat einschließt und der Luftdrucksensor (220) auf der zweiten starren Wand (2122) angeordnet ist.

5. Sensorvorrichtung nach Anspruch 4, wobei die erste starre Wand (2121) in einer Form eines Bogens oder einer U-Form ist und die erste starre Wand (2121) auf das Substrat aufgerastet ist, um die rohrförmige Struktur zu bilden.

6. Sensorvorrichtung nach Anspruch 4, wobei der Elastizitätsmodul der ersten starren Wand (2121) größer als 10 GPa ist.

7. Sensorvorrichtung nach Anspruch 1, wobei die zwei elastischen Wände (211) aus Elastomeren gebildet sind, die jeweils an den zwei Enden der rohrförmigen Struktur angeordnet und einander gegenüberliegend eingerichtet sind.

8. Sensorvorrichtung nach Anspruch 7, wobei der Elastizitätsmodul jedes der Elastomere in einem Bereich von 10 kPa - 10 MPa liegt.

9. Sensorvorrichtung nach Anspruch 7, weiter umfassend einen verformungsbegrenzenden Abschnitt (240), der so konfiguriert ist, dass er Verformung einer zweiten Oberfläche (232) der Elastomere in mindestens einer Richtung begrenzt, wobei die zweite Oberfläche (232) der Elastomere mit dem verformungsbegrenzenden Abschnitt (240) in Kontakt steht.

10. Sensorvorrichtung nach Anspruch 9, wobei der Elastizitätsmodul des verformungsbegrenzenden Abschnitts (240) größer als der Elastizitätsmodul jeder der elastischen Wände (211) ist und kleiner als der Elastizitätsmodul der starren Wand ist.

11. Sensorvorrichtung nach Anspruch 10, wobei der Elastizitätsmodul des verformungsbegrenzenden Abschnitts (240) in einem Bereich von 0,5 GPa - 10 GPa liegt.

12. Sensorvorrichtung nach Anspruch 9, wobei der verformungsbegrenzende Abschnitt (240) so konfiguriert ist, dass er Verformung der zweiten Oberfläche (232) der Elastomere, bevor sie gedehnt oder komprimiert werden, in einer ersten Richtung begrenzt; und sich der verformungsbegrenzende Abschnitt (240) in einer Ausrichtungsrichtung der zwei elastischen Wände (211) erstreckt, wobei die erste Richtung eine Ausdehnungsrichtung des verformungsbegrenzenden Abschnitts (240) ist.

13. Sensorvorrichtung nach einem der Ansprüche 9-12, wobei der verformungsbegrenzende Abschnitt (240) eine flexible Leiterplatte (241) einschließt, wobei die flexible Leiterplatte (241) auf einer Außenseite eines Substrats angeordnet ist und das Substrat und die Elastomere bedeckt.

14. Sensorvorrichtung nach einem der Ansprüche 1-8, weiter umfassend eine flexible Leiterplatte (241), wobei die flexible Leiterplatte (241) eine Außenseite der starren Wand bedeckt und eine zweite Oberfläche (232) aus Elastomeren nicht oder nur teilweise bedeckt.

15. Sensorvorrichtung nach Anspruch 14, wobei sich die flexible Leiterplatte (241) in einer Richtung erstreckt, die senkrecht zu einer Ausrichtungsrichtung der zwei elastischen Wände (211) verläuft.

## Revendications

1. Dispositif de détection comprenant une cavité (210) et un capteur de pression d'air (220), dans lequel le capteur de pression d'air (220) est disposé au sein de la cavité (210),
le dispositif de détection est **caractérisé en ce que** :
la cavité (210) inclut deux parois élastiques (211) disposées opposées l'une à l'autre et une paroi rigide (212) connectant les deux parois élastiques (211), dans lequel la paroi rigide (212) est sous la forme d'une structure tubulaire, et les deux parois élastiques (211) sont configurées pour sceller la structure tubulaire pour former la cavité (210) ;
dans lequel une déformation des deux parois élastiques (211) change la pression d'air au sein de la cavité (210), et le capteur de pression d'air (220) est configuré pour générer un signal électrique en réponse au changement de la pression d'air.

2. Dispositif de détection selon la revendication 1, dans lequel un rapport du module de Young de la paroi rigide (212) au module de Young de chacune des deux parois élastiques (211) est supérieur à 1 000.

3. Dispositif de détection selon la revendication 1, dans lequel un volume de la cavité (210) est dans une plage de 1 mm³ - 100 mm³.

4. Dispositif de détection selon la revendication 1, dans lequel la paroi rigide (212) inclut une première paroi rigide (2121) et une seconde paroi rigide (2122) qui sont connectées l'une à l'autre, la seconde paroi rigide (2122) inclut un substrat, et le capteur de pression d'air (220) est disposé sur la seconde paroi rigide (2122).

5. Dispositif de détection selon la revendication 4, dans lequel la première paroi rigide (2121) est sous la forme d'un arc ou d'un U, et la première paroi rigide (2121) est clipsée sur le substrat pour former la structure tubulaire.

6. Dispositif de détection selon la revendication 4, dans lequel le module de Young de la première paroi rigide (2121) est supérieur à 10 Gpa.

7. Dispositif de détection selon la revendication 1, dans lequel les deux parois élastiques (211) sont formées par des élastomères qui sont disposés à deux extrémités de la structure tubulaire, respectivement, et sont agencés opposés l'un à l'autre.

8. Dispositif de détection selon la revendication 7, dans lequel le module de Young de chacun des élastomères est dans une plage de 10 KPa-10 MPa.

9. Dispositif de détection selon la revendication 7, comprenant en outre une portion de limitation de la déformation (240) configurée pour limiter la déformation d'une seconde surface (232) des élastomères dans au moins une direction, la seconde surface (232) des élastomères est en contact avec la portion de limitation de la déformation (240).

10. Dispositif de détection selon la revendication 9, dans lequel le module de Young de la portion de limitation de la déformation (240) est supérieur au module de Young de chacune des parois élastiques (211) et est inférieur au module de Young de la paroi rigide.

11. Dispositif de détection selon la revendication 10, dans lequel le module de Young de la portion de limitation de la déformation (240) est dans une plage de 0,5 GPa-10 GPa.

12. Dispositif de détection selon la revendication 9, dans lequel la portion de limitation de la déformation (240) est configurée pour limiter la déformation de la seconde surface (232) des élastomères en étirement ou en compression dans une première direction ; et la portion de limitation de la déformation (240) s'étend dans une direction d'alignement des deux parois élastiques (211), la première direction étant une direction d'extension de la portion de limitation de la déformation (240).

13. Dispositif de détection selon l'une quelconque des revendications 9-12, dans lequel la portion de limitation de la déformation (240) inclut une carte de circuit flexible (241), la carte de circuit flexible (241) est disposée sur un côté extérieur d'un substrat et couvre le substrat et les élastomères.

14. Dispositif de détection selon l'une quelconque des revendications 1-8, comprenant en outre une carte de circuit flexible (241), dans lequel la carte de circuit flexible (241) couvre un côté extérieur de la paroi rigide et ne couvre pas ou couvre partiellement une seconde surface (232) d'élastomères.

15. Dispositif de détection selon la revendication 14, dans lequel la carte de circuit flexible (241) s'étend dans une direction perpendiculaire à une direction d'alignement des deux parois élastiques (211).
